# EUROPÄISCHE PATENTSCHRIFT

(11) **EP 2 411 531 B1**
(45) Veröffentlichungstag und Bekanntmachung des Hinweises auf die Patenterteilung: **27.12.2017**
(21) Anmeldenummer: 10705346.4
(22) Anmeldetag: 23.02.2010
(51) Int. Cl.: C12Q 1/68

(54) **VERFAHREN ZUR BESTIMMUNG DER PRÄDISPOSITION EINES PATIENTEN ZU VERÄNDERTER BIOTRANSFORMATION UND ZUR ENTWICKLUNG VON UNERWÜNSCHTEN ARZNEIMITTELWIRKUNGEN BEI EINER BEHANDLUNG DES PATIENTEN MIT ATORVASTATIN**
METHOD FOR DETERMINING THE PREDISPOSITION OF A PATIENT TO CHANGED BIOTRANSFORMATION AND TO THE DEVELOPMENT OF UNDESIRED PHARMACEUTICAL EFFECTS IN A TREATMENT OF THE PATIENT WITH ATORVASTATIN
PROCÉDÉ DE DÉTERMINATION DE LA PRÉDISPOSITION D'UN PATIENT À UNE BIOTRANSFORMATION MODIFIÉE ET AU DÉVELOPPEMENT D'EFFETS SECONDAIRES INDÉSIRABLES LORS D'UN TRAITEMENT PAR ATORVASTATIN

(30) Priorität: 26.03.2009 DE 102009015978
(43) Veröffentlichungstag der Anmeldung: 01.02.2012
(73) Patentinhaber: Robert Bosch Gesellschaft für medizinische Forschung mbH, 70376 Stuttgart (DE)
(72) Erfinder: KLEIN, Kathrin, 70197 Stuttgart (DE); RIEDMAIER, Stephan, 87770 Oberschönegg (DE); ZANGER, Ulrich, 70825 Korntal (DE)
(74) Vertreter: Bee, Joachim
(86) Internationale Anmeldenummer: PCT/EP2010/052245
(87) Internationale Veröffentlichungsnummer: WO 2010/108737

(56) Entgegenhaltungen:
- WO-A1-2008/032921
- US-B1- 6 528 260
- US-B2- 6 479 236
- WILKE RUSSELL A ET AL: "RELATIVE IMPACT OF CYP3A GENOTYPE AND CONCOMITANT MEDICATION ON THE SEVERITY OF ATORVASTATIN-INDUCED MUSCLE DAMAGE" PHARMACOGENETICS AND GENOMICS, LIPPINCOTT WILLIAMS & WILKINS, PHILADELPHIA, PA, US, Bd. 15, Nr. 6, 1. Juni 2005 (2005-06-01), Seiten 415-421, XP008077717 ISSN: 1744-6872
- CHEN Y ET AL.: "Genetic variants of human UGT1A3: Functional characterization and frequency distribution in a Chinese Han population" DRUG METABLISM AND DISPOSITION, Bd. 34, Nr. 9, 2006, Seiten 1462-1467, XP002582410 in der Anmeldung erwähnt
- SKOTTHEIM I B ET AL: "Statin induced myotoxicity: The lactone forms are more potent than the acid forms in human skeletal muscle cells in vitro" EUROPEAN JOURNAL OF PHARMACEUTICAL SCIENCES, ELSEVIER, AMSTERDAM, NL LNKD- DOI:10.1016/J.EJPS.2007.12.009, Bd. 33, Nr. 4-5, 23. April 2008 (2008-04-23), Seiten 317-325, XP022586184 ISSN: 0928-0987 [gefunden am 2008-01-18]
- RIEDMAIER S ET AL.: "UDP-Glucuronosyltransferase (UGT) polymorphisms affect Atorvastatin lactonization in vitro and in vivo" CLINICAL PHARMACOLOGY & THERAPEUTICS, Bd. 87, Nr. 1, Januar 2010 (2010-01), Seiten 65-73, XP002582411

### SEQUENZPROTOKOLL

<110> Robert Bosch Gesellschaft für medizinische Forschung mbH
<120> Verfahren zur Bestimmung der Prädisposition eines Patienten zu veränderter Biotransformation und zur Entwicklung von unerwünschten Arzneimittelwirkungen bei einer Behandlung des Patienten mit Atorvastatin
<130> 2637P101
<160> 34
<170> PatentIn version 3.3
<210> 1
   <211> 30
   <212> DNA
   <213> Künstliche Sequenz
<220>
   <223> Amplifikationsprimer
<400> 1
   acgttggatg cctggatgac tgaaataaag 30
<210> 2
   <211> 30
   <212> DNA
   <213> Künstliche Sequenz
<220>
   <223> Amplifikationsprimer
<400> 2
   acgttggatg cagcgtggag gctggctatg 30
<210> 3
   <211> 30
   <212> DNA
   <213> Künstliche Sequenz
<220>
   <223> Amplifikationsprimer
<400> 3
   acgttggatg acttggatgt tccccagagt 30
<210> 4
   <211> 30
   <212> DNA
   <213> Künstliche Sequenz
<220>
   <223> Amplifikationsprimer
<400> 4
   acgttggatg cctctggggt gaggaccact 30
<210> 5
   <211> 30
   <212> DNA
   <213> Künstliche Sequenz
<220>
   <223> Amplifikationsprimer
<400> 5
   acgttggatg tgcacatcaa agaagagaac 30
<210> 6
   <211> 30
   <212> DNA
   <213> Künstliche Sequenz
<220>
   <223> Amplifikationsprimer
<400> 6
   acgttggatg acagatgcat gactgagaat 30
<210> 7
   <211> 30
   <212> DNA
   <213> Künstliche Sequenz
<220>
   <223> Amplifikationsprimer
<400> 7
   acgttggatg tgatggacta ccccaggcca 30
<210> 8
   <211> 30
   <212> DNA
   <213> Künstliche Sequenz
<220>
   <223> Amplifikationsprimer
<400> 8
   acgttggatg ctgaaggcta ttatgacaag 30
<210> 9
   <211> 15
   <212> DNA
   <213> Künstliche Sequenz
<220>
   <223> Extensionsprimer
<400> 9
   ctccctgaac ccacc 15
<210> 10
   <211> 17
   <212> DNA
   <213> Künstliche Sequenz
<220>
   <223> Extensionsprimer
<400> 10
   caagacaacc ctagcaa 17
<210> 11
   <211> 21
   <212> DNA
   <213> Künstliche Sequenz
<220>
   <223> Extensionsprimer
<400> 11
   ggatatttct tgtaaggatc a 21
<210> 12
   <211> 17
   <212> DNA
   <213> Künstliche Sequenz
<220>
   <223> Extensionsprimer
<400> 12
   tggttttggt cgttttt 17
<210> 13
   <211> 18
   <212> DNA
   <213> Künstliche Sequenz
<220>
   <223> Extensionsprimer
<400> 13
   cctggaaaag accgatca 18
<210> 14
   <211> 19
   <212> DNA
   <213> Künstliche Sequenz
<220>
   <223> Extensionsprimer
<400> 14
   tgctacattt gctttcttc 19
<210> 15
   <211> 21
   <212> DNA
   <213> Künstliche Sequenz
<220>
   <223> Extensionsprimer
<400> 15
   ctgagatggc cacaggactc c 21
<210> 16
   <211> 16
   <212> DNA
   <213> Künstliche Sequenz
<220>
   <223> Extensionsprimer
<400> 16 agtcctgtgg ccagcc 16
<210> 17
   <211> 17
   <212> DNA
   <213> Künstliche Sequenz
<220>
   <223> Extensionsprimer
<400> 17
   accaacacct ttccact 17
<210> 18
   <211> 18
   <212> DNA
   <213> Künstliche sequenz
<220>
   <223> Extensionsprimer
<400> 18
   gcatggagct cccgcaag 18
<210> 19
   <211> 16
   <212> DNA
   <213> Künstliche Sequenz
<220>
   <223> Extensionsprimer
<400> 19
   acgaaatggc ataggt 16
<210> 20
   <211> 19
   <212> DNA
   <213> Künstliche Sequenz
<220>
   <223> Extensionsprimer
<400> 20
   attgccatac ttctgaaaa 19
<210> 21
   <211> 20
   <212> DNA
   <213> Künstliche Sequenz
<220>
   <223> Extensionsprimer
<400> 21
   gacatattgt tcaacattgc 20
<210> 22
   <211> 15
   <212> DNA
   <213> Künstliche Sequenz
<220>
   <223> Extensionsprimer
<400> 22
   ccgttaacct ctgcg 15
<210> 23
   <211> 22
   <212> DNA
   <213> Künstliche Sequenz
<220>
   <223> Extensionsprimer
<400> 23
   tcgacaggta cttagccagc ac 22
<210> 24
   <211> 21
   <212> DNA
   <213> Künstliche Sequenz
<220>
   <223> Extensionsprimer
<400> 24
   gattcctact gtgttttttt t 21
<210> 25
   <211> 17
   <212> DNA
   <213> Künstliche Sequenz
<220>
   <223> Extensionsprimer
<400> 25
   aggaacattc catgtga 17
<210> 26
   <211> 26
   <212> DNA
   <213> Künstliche Sequenz
<220>
   <223> Extensionsprimer
<400> 26
   caaccaattc agaccacatg acattc 26
<210> 27
   <211> 15
   <212> DNA
   <213> Künstliche Sequenz
<220>
   <223> Extensionsprimer
<400> 27
   taccccaggc caatc 15
<210> 28
   <211> 25
   <212> DNA
   <213> Künstliche Sequenz
<220>
   <223> Amplifikationsprimer für die quantitative PCR
<400> 28
   tgttgaacaa tatgtctttg gtcta 25
<210> 29
   <211> 21
   <212> DNA
   <213> Künstliche Sequenz
<220>
   <223> Amplifikationsprimer für die quantitative PCR
<400> 29
   gaaggaattt gatcgcgtta c 21
<210> 30
   <211> 19
   <212> DNA
   <213> Künstliche Sequenz
<220>
   <223> Amplifikationsprimer für die quantitative PCR
<400> 30
   ggtggtggtc ctcaccctg 19
<210> 31
   <211> 21
   <212> DNA
   <213> Künstliche Sequenz
<220>
   <223> Amplifikationsprimer für die quantitative PCR
<400> 31
   gttcgcaaga ttcgatggtc g 21
<210> 32
   <211> 26
   <212> DNA
   <213> Künstliche Sequenz
<220>
   <223> Amplifikationsprimer für die quantitative PCR
<400> 32
   ggatattctc agtcatgcat ctgtgt 26
<210> 33
   <211> 18
   <212> DNA
   <213> Künstliche Sequenz
<220>
   <223> Amplifikationsprimer für die qualitative PCR
<400> 33
   ttcgatggtc gggttcca 18
<210> 34
   <211> 13
   <212> DNA
   <213> Künstliche Sequenz
<220>
   <223> Amplifikationsprimer für die quantitative PCR
<220>
   <221> modifizierte_Base
   <222> (1)..(1)
   <223> 6-Carboxyfluorescein
<220>
   <221> modifizierte_Base
   <222> (13)..(13)
   <223> Minor Groove Binder
<400> 34
   ccccaggcca atc 13

## Beschreibung

Die vorliegende Erfindung betrifft ein Verfahren zur Bestimmung einer Prädisposition eines Patienten zu veränderter Biotransformation und zur Entwicklung von unerwünschten Arzneimittelwirkungen bei der Behandlung des Patienten mit Statinen als Folge einer genetisch determinierten Änderung der Fähigkeit zur Biotransformation derselben.

Muskelerkrankungen, wie beispielsweise Myopathien und Rhabdomyelosen, stellen Erkrankungen der Muskeln dar, die beispielsweise durch die Gabe von Statinen ausgelöst werden können.

Statine, zu welchen der Wirkstoff Atorvastatin gezählt wird, sind Arzneistoffe, die 3-Hydroxy-3-methylglutaryl-Coenzym-A-Reductase-(HMG-CoA-Reductase)- Inhibitoren darstellen. HMG-CoA wiederum ist ein Zwischenprodukt der menschlichen Cholesterinsynthese, weshalb Statine hauptsächlich bei Fettstoffwechselstörungen als Cholesterinsenker eingesetzt werden. Dabei bewirken die Statine durch die Hemmung der HMG-CoA-Reductase eine Lipidsenkung im Blut. Da HMG-CoA ein an der Biosynthese von Cholesterin beteiligter Stoff ist, wird unter der Einwirkung von Statinen weniger Cholesterin im Körper gebildet als ohne die Gabe von Statinen. Zu den Vertretern der Statine zählen u.a. Atorvastatin, Cerivastatin, Fluvastatin, Lovastatin, Pravastatin, Rosuvastatin und Simvastatin.

Obwohl Statine gemeinhin als nützliche Arzneimittel gelten, bestehen Probleme bei der Therapie, nämlich zum Einen hinsichtlich der Unsicherheit in der Vorhersage der einer bestimmten Dosis entsprechenden Wirkung, und zum Anderen in dem Risiko der Entwicklung unerwünschter Arzneimittelwirkungen (vorliegend auch mit "UAW" abgekürzt, und gemeinhin auch als Nebenwirkungen bezeichnet).

Alle Statine, auch Atorvastatin, können unerwünschte Arzneimittelwirkungen hervorrufen, zu deren schwerwiegendsten die sog. toxischen Myopathien zählen, bei denen es sich um strukturelle und funktionelle Veränderungen der Skelettmuskulatur handelt. Die schwerste Form einer toxischen Myopathie ist die Rhabdomyelose, die sich u.a. in einer vollständigen Lähmung aller vier Gliedmaßen äußern und häufig tödlich verlaufen kann. In der Literatur sind bis zum Jahr 2003 ca. 3350 Fälle einer durch Lipidsenker ausgelösten Rhabdomyelose beschrieben worden.

Die Wirkstärke der verschiedenen, gegenwärtig auf dem Markt erhältlichen Statine ist dabei unterschiedlich; so weist beispielsweise Fluvastatin eine geringe Myopathiehäufigkeit auf, zeigt jedoch andererseits auch in der Höchstdosis nur eine der schwächsten lipidsenkenden Wirkungen.

Als weitere unerwünschte Arzneimittelwirkungen bei Einnahme von Statinen wie Atorvastatin wurden Leberschäden, ein Nachlassen der Gedächtnisleistung und der Aufmerksamkeit, sowie erhöhte Aggressivität und erhöhte Reizbarkeit beobachtet, sowie Kopfschmerz, Übelkeit, Blutarmut, Nervenschädigung, Haarausfall, etc.

Da nicht sämtliche Patienten, die einer Behandlung mit Statinen, insbesondere mit Atorvastatin, zur Senkung des Cholesteringehalts unterzogen werden, unerwünschte Arzneimittelwirkungen entwickeln, und Patienten unterschiedlich auf bestimmte Statine, insbesondere Atorvastatin, und deren Dosen reagieren, wäre es wünschenswert, im Vorfeld einer Statin-Therapie, insbesondere einer Atorvastatin-Therapie, die Prädisposition eines Patienten, unerwünschte Arzneimittelwirkungen zu entwickeln, oder anders als gewünscht auf die Therapie zu reagieren, bestimmen zu können.

Aufgabe der vorliegenden Erfindung ist es daher, ein Verfahren zur Bestimmung einer Prädisposition eines Patienten für die Entwicklung von unerwünschten Arzneimittelwirkungen bzw. für eine veränderte Wirksamkeit bei einer Behandlung des Patienten mit Statinen bereit zu stellen.

Gemäß der vorliegenden Erfindung wird diese Aufgabe dadurch gelöst, dass in einer biologischen Probe des Patienten das Vorliegen von zumindest einem Einzelnucleotid-Polymorphismus (SNP) in einem der folgenden Haplotypen des UGT1A3-Gens (Uridindiphosphat-Glucuronosyltransferase-Gen 1A3) bestimmt wird: UGT1A3*2 mit Varianten *2a, *2c, *2d, UGT1A3*3 oder UGT1A3*6, und/oder eine erhöhte UGT1A3-Genexpression bestimmt wird.

Die der Erfindung zugrunde liegende Aufgabe wird auf diese Weise vollkommen gelöst.

Die Erfinder konnten in eigenen Versuchen anhand der Untersuchung zahlreicher menschlicher Leberproben zeigen, dass genetische Variationen im UGT1A3-Gen zu einer erhöhten UGT1A3-Genexpression führten. Ferner konnten die Erfinder zeigen, dass die erhöhte Genexpression mit einer erhöhten Lactonisierung des Statins Atorvastatin (ATV) einherging.
Ein erhöhter Gehalt an ATV-Lacton findet sich bei Atorvastatin-Patienten, die unter einer Myopathie leiden, ebenso wie erhöhte Konzentrationen an Hydroxy-Metaboliten von Atorvastatin (siehe Hermann et al., "Exposure of atorvastatin is unchanged but lacton and acid metabolite are increased several-fold in patients with atorvastatin-induced myopathy", Clin. Pharmacol. Ther., 2006, 79:532 - 539). Zudem wurde an einem Zellkulturmodell nachgewiesen, dass Statin-Lactone eine im Vergleich zu den jeweiligen Statinsäuren um 14-37-fach erhöhte Myotoxizität aufwiesen (Skottheim et al., Statin induced myotoxicity: the lactone forms are more potent than the acid forms in human sceletal muscle cells in vitro; Eur. J. Pharm. Sci. 33:317-25 (2008)).
Bekannt ist, dass Atorvastatin *in vivo* unter anderem in 2-(ortho-) und 4-(para-)-Hydroxy-ATV-Säuren (pharmakologisch wirksame Metabolite) biotransformiert wird. Alternativ kann die freie Säureseitenkette in zyklisches ATV-Lacton umgewandelt werden. Aufgrund der höheren Lipophilie des ATV-Lactons wird dieses viel eher hydroxyliert als ATV selber (siehe Jacobsen et al., Drug Metabol. Dispos. 28(11):1369-78 (2000). Die Erfinder konnten mit den vorliegenden Erkenntnissen somit erstmals zeigen, dass der erhöhte Gehalt an ATV-Lacton und an Hydroxy-ATV-Lacton auf eine erhöhte Aktivität des Enzyms Uridindiphosphat-Glucuronosyltransferase, bzw. vielmehr auf die durch die genetischen Variationen ausgelöste erhöhte Aktivität der Isoform 1A3 dieses Enzyms, zurückzuführen ist.
Die ATV-Lactonisierung kann zwar durch mehrere UGT-Isoformen katalysiert werden (siehe bspw. Goosen et al.: "Atorvastatin glucoronidation is minimally and nonselectively inhibited by the Fibrates Gemfibrozil, Fenofibrate, and Fenofibric Acid", Am. Soc. Clinic. Pharma. Therap., 2007: 35 (8) 1315 - 1323), allerdings war bisher nicht bekannt, welche der Isoformen *in vivo* die Hauptfunktion einnimmt. Wie bereits erwähnt, sind UGTs (UDP-Glucoronosyltransferasen) Enzyme, die u.a. die Lactonisierung von Statinen, bspw. Atorvastatin bewirken. Die lactonisierten Statine wiederum werden - gegenüber den Statinen selber - bevorzugt von dem Enzym CYP3A in Hydroxy-Statin-Lactone umgewandelt. So offenbarten Wilke et al. ("Relative impact of CYP3A genotype and concomitant meidcation on the severity of Atorvastatin-induced muscle damage" einen Zusammenhang zwischen der Entwicklung von Muskelerkrkankungen und veränderter Biotransformation bei der Behandlung mit Atorvastatin, wobei der Focus hier auf der Expression bestimmter CYP3A-Gene lag.
Unter dem UGT1A3-Gen wird vorliegend stets die kodierende Sequenz dieses Gens sowie die Intron-Sequenzen und die 5'- und 3'-untranslatierten/regulatorischen Regionen des Gens verstanden.
Mit dem nun vorliegenden Verfahren ist es erstmals möglich, Patienten, die einer Statin-Behandlung unterzogen werden sollen, insbesondere einer Atorvastatin-Behandlung, bzw. Patienten, die bereits einer Statin-Behandlung unterzogen werden, dahingehend zu screenen, ob sie genetisch zu einer höheren Aktivität des Isozyms UGT1A3 veranlagt sind, und dadurch Gefahr laufen, vermehrt Statin-Lacton und Hydroxy-Statin-Lacton zu bilden, was zu den erwähnten Muskelerkrankungen führen kann, oder zu einem teilweisen Therapieversagen, da die Statin-Lactone pharmakologisch unwirksame Metabolite darstellen. Atorvastatin besitzt demnach in diesem Fall bzw. bei diesen Patienten mit großer Wahrscheinlichkeit nicht dieselbe Wirksamkeit, wie es bei Patienten der Fall ist, die diese Polymorphismen nicht aufweisen. Vorteilhafterweise kann durch die Bestimmung der Polymorphismen dann gerade vorhergesagt werden, ob Atorvastatin in erhöhtem Maße biotransformiert wird und damit in seiner Dosierung unwirksamer sein wird als bei einem Patienten, der die Polymorphismen nicht aufweist; wenn Polymorphismen gemäß der vorliegenden Erfindung identifiziert werden, kann dann entweder die tatsächliche oder geplante Dosierung von Atorvastatin entsprechend angepasst, also erhöht werden, um eine ähnliche Wirksamkeit von Atorvastatin wie bei Wildtyp-Patienten zu erreichen, bzw. auf ein anderes Statin oder einen anderen Therapieansatz zurückgegriffen werden, um unerwünschte Arzneimittelwirkungen zu vermeiden.
Gemäß der vorliegenden Erfindung wird dies durch die Bestimmung von zumindest einem SNP in einem der folgenden Haplotypenim UGT1A3-Gen vorgenommen: UGT1A3*2 mit Varianten *2a, *2c, *2d, UGT1A3*3 oder UGT1A3*6. Dadurch kann die Therapie des mit dem Statin zu behandelnden Patienten individuell abgestimmt werden, also entweder eine gänzlich andere Alternative zu den Statinen eingesetzt werden, oder aber die Dosierung und damit die Wirksamkeit von bestimmten zu verabreichenden Statinen auf den Patienten individuell berücksichtigt werden.

Neben Atorvastatin werden gegenwärtig auch andere Statine eingesetzt, wie bspw. Cerivastatin, Fluvastatin, Lovastatin, Pravastatin, Rosuvastatin und Simvastatin.

Die genannten Statine werden im Markt gegenwärtig bspw. unter den Bezeichnungen Sortis®, Lipitor® (Atorvastatin), Baycol®, Zenas® (Cerivastatin), Cranoc®, Lescol®, Locol®, Fractal® (Fluvastatin), Mevinacor® (Lovastatin), Mevalotin®, Pravasin®, Pravachol® (Pravastatin), Crestor® (Rosuvastatin), Gerosim®, Simvabeta®, Zocor® (Simvastatin) und deren jeweilige Generika vertrieben.

Mit dem nunmehr vorliegenden Verfahren kann vorteilhaft eine gezielte und individualisierte Cholesterinsynthesehemmer-Therapie bereitgestellt werden, wodurch die Patienten, die einer solchen Therapie unterzogen werden müssen, entweder vor der Behandlung mit einem der genannten Arzneimitteln oder während, auf das Vorliegen von SNPs im UGT1A3-Gen untersucht werden. Liegt ein SNP vor, der zu einer erhöhten Expression dieses Gens und damit zu einer erhöhten Aktivität der UDP-Glucuronyltransferase führt, kann die Behandlung entweder mit Alternativen zu den Statinen oder aber mit einem anderen als dem vorgesehenen Statin durch- bzw. weitergeführt werden, oder aber mit einer anderen Dosierung als die ursprüngliche bzw. ursprünglich vorgesehene.

Erfindungsgemäß wird zumindest einer der folgenden Haplotypen bestimmt: UGT1A3*2, UGT1A3*3, UGT1A3*6, und insbesondere einer der folgenden SNPs im UGT1A3-Gen:
UGT1A3*2: rs55772651, rs1983023, rs56304713, rs45507691, rs3806597, rs3806596, rs3821242, rs6706232, rs6431625, rs17868336, rs7574296;
UGT1A3*3: rs55772651, rs56304713, rs3806597, rs3806596, rs3821242, rs6706232, rs7574296
UGT1A3*6: rs55772651, rs1983023, rs56304713, rs3806597, rs3806596, rs3821242, rs6706232, rs6431625, rs7574296, rs45449995

Mit dem Ausdruck "genomische Pos. in AF297093" ist vorliegend ein SNP bezeichnet, für das keine rs-Nummer verfügbar ist, dessen Position in dem UGT1 Gen-Locus, der gemäß der öffentlich zugänglichen Datenbanken mit der Zugangsnummer AF297093 bezeichnet ist, entsprechend angegeben ist. (Zum UGT1-Gen-Locus siehe bspw. die EMBL-EBI-Datenbank unter http://www.ebi.ac.uk/cgi-bin/expasyfetch?AF297093 oder die Datenbank GenBank des National Center for Biotechnology Information NCBI http://www.ncbi.nlm.nih.gov/)

Mit SNPs (engl. Single Nucleotide Polymorphisms) werden Variationen von einzelnen Basenpaaren in einem DNA-Strang im Vergleich zum Wildtyp in einer bestimmten Population bezeichnet. SNPs stellen ca. 90 % aller genetischen Varianten im menschlichen Genom dar, und treten ungleichmäßig stark an bestimmten Regionen im Genom auf. Sie stellen Mutationen dar, d. h. genetische Veränderungen, die sich zu einem gewissen Grad im Genpool einer Population durchgesetzt haben. Dabei können die SNPs als Substitutionen auftreten, bei welchen eine Base, bspw. Cytosin, gegen eine andere Base, bspw. Thymin, ausgetauscht ist, oder aber als Deletionen oder Insertionen.

SNPs weisen dabei immer einen von zwei oder sehr selten auch mehreren Zuständen auf und werden allelisch vererbt. Die Mehrzahl der bekannten SNPs betrifft nicht codierende Bereiche im Genom, d.h. Regionen die entweder zwischen Genen oder zwischen Exonbereichen einzelner Gene liegen. Grundsätzlich können diese Genvarianten in nicht- codierenden Bereichen auch regulatorische Sequenzen, z.B. Promotoren, Enhancer oder Spleißstellen betreffen und damit Auswirkungen auf die Expression von Genen haben. SNPs, die direkt die kodierende Sequenz betreffen, können still sein, d.h. der Basenaustausch verändert nicht die Übersetzung des entsprechenden Triplettcodes in die analoge Aminosäure und hat somit also keinen Einfluss auf die Peptidsequenz. Allerdings können sich aus unterschiedlicher Häufigkeit äquivalenter t-RNAs für spezifische Basentripletts Unterschiede für die Effizient der Translation ergeben und somit kann die Expression bestimmter Gene post-transkriptionell durch stille SNPs beeinflusst werden. Einige SNPs haben codierende Funktion, d.h. die unterschiedlichen Allele führen zum Einbau einer unterschiedlichen Aminosäure in das entstehende Peptid, mit der Folge, dass dessen Funktion verändert sein kann.

Im Genom können SNPs, wenn sie bi-allelisch vorliegen in drei möglichen Genotypen vorkommen, nämlich in einer von zwei möglichen homozygoten Formen (Allel 1/ Allel 1 oder Allel 2 / Allel 2) oder aber in einer heterozygoten Form (Allel 1/ Allel 2). Benachbarte SNPs können in unterschiedlichem Ausmaß miteinander gelinkt sein. D.h., zu einem gewissen Prozentsatz treten sie in der Bevölkerung in einer bestimmten Kombination nur gemeinsam auf und bilden damit einen sogenannten Haplotyp. Unter "Kopplung" versteht man dabei die Tendenz, dass die jeweils an zwei verschiedenen Positionen auf einem Chromosom vorhandenen Allele gemeinsam (auf demselben Chromosom) weitergegeben, d.h. gekoppelt vererbt, werden. Dabei spricht man allgemein von einem "Kopplungsungleichgewicht" (engl.: "Linkage Disequilibrium") bei Allelen, wenn diese dazu tendieren, gemeinsam vererbt zu werden.

Da genomische DNA doppelsträngig ist, kann jeder SNP bezüglich jedes der beiden Stränge identifiziert werden. Die in der vorliegenden Anmeldung bevorzugten SNPs beinhalten zwar eine Substitution eines Nuclotids durch ein anderes an der polymorphen Stellen des SNPs, aber SNps können auch komplexer sein und können eine Deletion eines Nucleotids aus einer, oder eine Insertion eines Nucleotids in eine, von zwei korrespondierenden Sequenzen aufweisen.

Der Ausdruck "bestimmen", wie er vorliegend für die Bestimmung der SNPs benutzt wird, bezieht verschiedene Methoden und Verfahren zur Analyse eines oder mehrerer SNP an einer bestimmten Stelle im Genom, wobei der Ausdruck sowohl die direkte Bestimmung, d.h. bspw. die Sequenzierung, als auch die indirekte Bestimmung, also bspw. die Amplifizierung und/oder Hybridisierung, mit einschließt.

Die Erfinder haben nun erkannt, dass es überraschenderweise möglich ist, anhand bestimmter SNPs im UGT1A3-Gen eine genetische Prädisposition für die Entwicklung von Muskelerkrankungen oder einer veränderten Wirksamkeit bei der Verabreichung von Statinen zu diagnostizieren.

Mit dem neuen Verfahren ist es nun erstmals möglich, eine individuell wahrscheinliche Ausprägung einer Muskelerkrankung bei der Verabreichung von Atorvastatin, bzw. eine individuell wahrscheinliche erniedrigte Wirksamkeit von Atorvastatin zu prognostizieren.

Dabei ist in einer weiteren Ausführungsform bevorzugt, wenn der zumindest eine SNP ausgewählt ist aus den mit den SNPs der UGT1A3*2, UGT1A3*3, und UGT1A3*6 Haplotypen in Kopplungsungleichgewicht befindlichen SNPs.

Dies bedeutet, dass auch SNPs im Rahmen des erfindungsgemäßen Verfahrens nachgewiesen werden können, die ebenfalls als Marker der Haplotypen der UGT1A3*2, UGT1A3*3, und UGT1A3*6 verwendet werden können, aber hier nicht explizit aufgeführt sind, die sich aber mit den eben genannten SNPs im Kopplungsungleichgewicht befinden (siehe bspw. Ménard V. et al., "Analysis of inherited genetic variations at the UGT1 locus in the French-Canadian population." Hum Mutat. 2009 Feb 8).

In einer weiteren Ausführungsform des erfindungsgemäßen Verfahrens ist bevorzugt, wenn die erhöhte UGT1A3-Genexpression über einen erhöhten mRNA-Level und/oder einen erhöhten Protein-Level bestimmt wird.

Der durch die Erfinder erbrachte Nachweis, dass die genetischen Variationen im UGT1A3-Gen zu einer erhöhten UGT1A3-Expression und damit auch zu einer erhöhten Aktivität dieses Enzyms führen, steht konträr zu den bisher - allerdings lediglich mittels rekombinanter Isoenzyme - gewonnenen Erkenntnissen, wonach die genetischen Varianten in der Regel mit einer erniedrigten Funktion assoziiert wurden (siehe bspw. Chen et al., "Genetic Variants of Human UGT1A3: Functional Characterization and Frequency Distribution in a Chinese Han Population", Drug Metabolism and Disposition 2006, 34:1462-1467; Caillier et al., "A pharmacogenomics Study of the human estrogen glucuronosyltransferase UGT1A3", Pharmacogenet. Genomics 2007, 17(7):481-95).

Daher sind die vorliegenden Erkenntnisse und das bereitgestellte Verfahren - obgleich UGT1A3 sowie UGT1A3-Polymorphismen im Stand der Technik zwar bereits identifiziert waren - neu und überraschend, da die Polymorphismen mit einer erniedrigten Aktivität oder Expression von UGT1A3 assoziiert waren. Die Erfinder der vorliegenden Anmeldung haben aber nun gerade herausgefunden, dass die Polymorphismen mit einer erhöhten UGT1A3-Expression und in der Folge auch erhöhten Aktivität einhergehen, was, wie weiter oben beschrieben, zu der vermehrten ATV-Lactonisierung führt.

Insbesondere ist bei dem erfindungsgemäßen Verfahren bevorzugt, wenn zum Nachweis des zumindest einen SNP im UGT1A3-Gen ein Oligonucleotid eingesetzt wird, das ausgewählt ist aus einem der in den Tabellen 1 und 2 aufgeführten Oligonucleotiden, bzw. aus den Oligonucleotiden mit den SEQ-ID Nr. 1 bis 27:

**Tabelle 1: UGT1A3 Amplifikationsprimer.**

| SEQ-ID Nr | genom. Position | Primer Sequenz (5'→ 3') | Ampl.-Produkt (bp) |
|---|---|---|---|
| 1 | 144852-145219 | ACGTTGGATGCCTGGATGACTGAAATAAAG | 388 |
| 2 | | ACGTTGGATGCAGCGTGGAGGCTGGCTATG | |
| 3 | 145477-145927 | ACGTTGGATGACTTGGATGTTCCCCAGAGT | 471 |
| 4 | | ACGTTGGATGCCTCTGGGGTGAGGACCACT | |
| 5 | 145934-146495 | ACGTTGGATGTGCACATCAAAGAAGAGAAC | 582 |
| 6 | | ACGTTGGATGACAGATGCATGACTGAGAAT | |
| 7 | 146519-146741 | ACGTTGGATGTGATGGACTACCCCAGGCCA | 243 |
| 8 | | ACGTTGGATGCTGAAGGCTATTATGACAAG | |

Die Oligonucleotide mit den SEQ-ID Nrn. 1, 3, 5, 7 stellen dabei forward ("f") Primer dar, die Oligonucleotide mit den SEQ-ID Nrn. 2, 4, 6, und 8 reverse ("r") Primer.

**Tabelle 2: Extensionsprimer für die MALDI-TOF Massenspektrometrie Analyse**

| SEQ-ID Nr. | Assay | gen. Position | Primer Sequenz (5'→ 3') | Masse des Ampl.-Produktes (Da) |
|---|---|---|---|---|
| 9 | 1 | 144977 | CTCCCTGAACCCACC | 4417.9 |
| 10 | 2 | 144984 | CAAGACAACCCTAGCAA | 5141.4 |
| 11 | 1 | 145154 | GGATATTTCTTGTAAGGATCA | 6475.2 |
| 12 | 3 | 145182 | TGGTTTTGGTCGTTTTT | 5219.4 |
| 13 | 1 | 145531 | CCTGGAAAAGACCGATCA | 5501.6 |
| 14 | 1 | 145669 | TGCTACATTTGCTTTCTTC | 5710.7 |
| 15 | 3 | 145765 | CTGAGATGGCCACAGGACTCC | 6416.2 |
| 16 | 1 | 145751 | AGTCCTGTGGCCAGCC | 4858.2 |
| 17 | 1 | 145815 | ACCAACACCTTTCCACT | 5034.3 |
| 18 | 2 | 145874 | GCATGGAGCTCCCGCAAG | 5509.6 |
| 19 | 2 | 145968 | ACGAAATGGCATAGGT | 4954.3 |
| 20 | 3 | 146062 | ATTGCCATACTTCTGAAAA | 5770.8 |
| 21 | 1 | 146076 | GACATATTGTTCAACATTGC | 6091.0 |
| 22 | 3 | 146207 | CCGTTAACCTCTGCG | 4503.9 |
| 23 | 1 | 146211 | TCGACAGGTACTTAGCCAGCAC | 6704.4 |
| 24 | 1 | 146253 | GATTCCTACTGTGTTTTTTTT | 6374.2 |
| 25 | 1 | 146271 | AGGAACATTCCATGTGA | 5218.4 |
| 26 | 1 | 146356 | CAACCAATTCAGACCACATGACATTC | 7852.1 |
| 27 | 1 | 146542 | TACCCCAGGCCAATC | 4481.9 |

Genomische (gen.) Position entsprechend der Datei mit der GenBank Zugangsnummer (Acc. Number) AF297093.1.

Die Erfinder konnten mit diesen Oligonucleotiden erfolgreich die die SNPs enthaltenden Regionen amplifizieren sowie die SNPs über die Extension dieser Oligonucleotide (Primer) identifizieren. Daher betrifft die vorliegende Erfindung auch diese Oligonucleotide selber.

In einer weiteren Ausführungsform ist bevorzugt, wenn zur Bestimmung des zumindest einen SNP zumindest eines der folgenden Verfahren eingesetzt wird: PCRbasierte Verfahren, DNA-Sequenzierverfahren, Hybridisierungsverfahren, Massenspektroskopie, HPLC-Verfahren, Primer-Extension-Verfahren.

Diese Verfahren sind im Stand der Technik hinreichend bekannt und werden bereits zur Identifizierung von SNPs eingesetzt. Beispielhaft wird hierbei auf "Guide to Mutation Detection" von Graham R. Taylor und Ian N. M. Day (Wiley & Sons, 2005) verwiesen, in dem diese Verfahren hinlänglich beschrieben sind.

In einer weiteren Ausführungsform ist bevorzugt, wenn zur Amplifizierung der die SNP enthaltenden Regionen die folgenden PCR-Zyklen eingesetzt werden:

Denaturierung bei ca. 95°C für 15 min, gefolgt von 5 Zyklen mit den Schritten ca. 95°C für 20 sec, ca. 65°C für 30 sec und ca. 72°C für 1 min; gefolgt von 40 Zyklen mit den Schritten ca. 95°C für 20 sec, ca. 62°C für 30 sec und ca. 72°C für 1 min; gefolgt von einem finalen Extensionsschritt bei ca. 72°C für 10 min.

Ferner betrifft die Erfindung auch diagnostische Kits zur Bestimmung einer Prädisposition eines Patienten für die Entwicklung von Muskelerkrankungen bei einer Behandlung des Patienten mit Statinen, wobei das Kit zumindest ein Oligonucleotid aufweist, das ausgewählt ist aus zumindest einem der Oligonucleotide mit den SEQ-ID-Nrn. 1 bis 10, 13, 14, 15, 17 bis 21, sowie 24 bis 27 aus dem beigefügten Sequenzprotokoll.

Mit dem erfindungsgemäßen Kit wird ein einfaches Tool bereitgestellt, mit dem schnell zumindest ein entsprechendes SNP im UGT1A3 Gen und nachgewiesen werden kann, wodurch Patienten identifiziert werden können, die mit großer Wahrscheinlichkeit bei einer Therapie mit einem Statin Muskelerkrankungen als Nebenwirkung entwickeln werden.

Die Erfindung betrifft ferner die Verwendung von zumindest einem SNP im menschlichen UGT1A3-Gen, insbesondere die SNPs rs55772651, rs1983023, rs56304713, rs45507691, rs3806597, rs3806596, rs3821242, rs6706232, rs6431625, rs17868336, rs7574296, g.146356 (gen. Pos. in AF297093), rs45449995 sowie auch weiterer, mit den UGT1A3*2, UGT1A3*3, und UGT1A3*6 Haplotypen in Kopplungsungleichgewicht befindlichen SNPs zur Bestimmung der Prädisposition eines Patienten zur Entwicklung von Muskelerkrankungen bei der Behandlung von Statinen.

Weitere Vorteile und Merkmale der Erfindung ergeben sich aus der nachfolgenden Beschreibung und den beigefügten Figuren.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

In den Figuren zeigen:
- Fig. 1: eine Übersicht über die Biotransformation des Statins Atorvastatin im menschlichen Körper;
- Fig. 2: Diagramme bezüglich der Aktivitäten der verschiedenen UGT-Isoenzyme, wonach das Isoenzym 1A3 die höchste Aktivität hinsichtlich der ATV-Lactonisierung zeigt;
- Fig. 3: Diagramme zur Verteilung des UGT1A3 Proteins in Patientenproben und Korrelation der Aktivität der Atorvastatin-Lactonisierung (rechts) zum Proteingehalt in der menschlichen Leber;
- Fig. 4: eine schematische Übersicht über die Struktur der UGT1A3 Haplotypen in der menschlichen Leber;
- Fig. 5: die Korrelation Genotyp - Phänotyp für UGT1A3 - mRNA
- Fig. 6: die Korrelation Genotyp - Phänotyp für UGT1A3 - Protein
- Fig. 7: die Korrelation Genotyp - Phänotyp für UGT1A3 - Lactonisierung-Aktivitäten

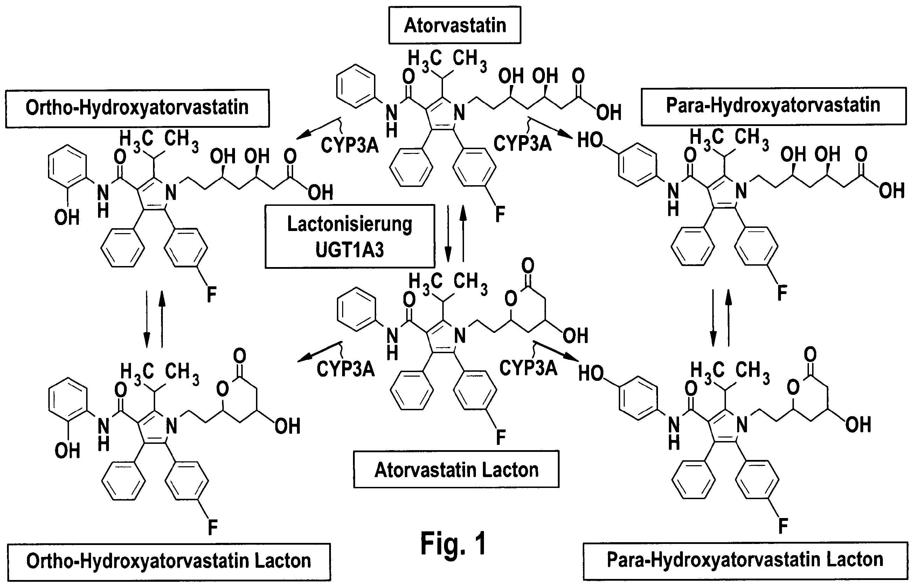

Fig. 1 zeigt eine schematische Übersicht über den Metabolismus des Statins Atorvastatin, dessen Strukturformel unterhalb des Namens angezeigt ist. "HMGCR" ist die Abkürzung für das Enzym 3-Hydroxy-3-methylglutaryl-Coenzym-A-Reductase-(HMG-CoA-Reductase), das durch die Statine gehemmt wird.

Dem Schema ist zu entnehmen, dass das Enzym UGT (und - wie die Erfinder nachweisen konnten - überwiegend UGT1A3), verantwortlich ist für die Lactonisierung von Atorvastatin (ATV) zu ATV-Lacton (unterer Schema-Rand, mittig).

Ferner ist in dem Schema dargestellt, dass Enzyme der Cytochrom P450 Subfamilie CYP3A (das sind v.a. CYP3A4 und CYP3A5) die Hydroxylierung von Atorvastatin entweder in 2- oder 4- Hydroxy-Atorvastatin katalysiert, allerdings weniger effektiv als die Hydroxylierung von ATV-Lacton (verdeutlicht durch die unterschiedliche Dicke der die Reaktion verdeutlichenden Pfeile).

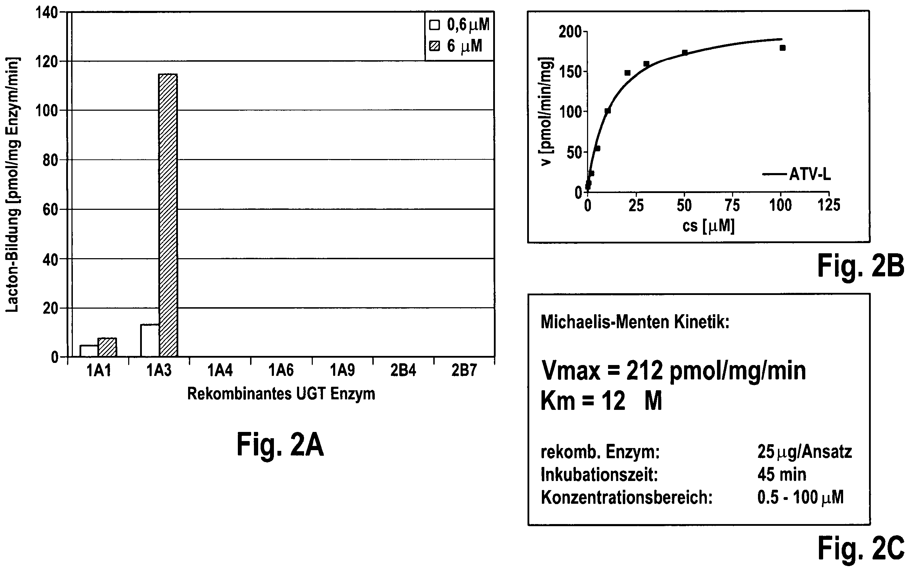

Fig. 2 zeigt in einem Säulendiagramm (links; 2A) die gemessenen Enzymaktivitäten der verschiedenen rekombinanten UGT-Isoenzyme bei zwei unterschiedlichen Substratkonzentrationen (0,6µM, weiße Balken, und 6 µM, schwarze Balken). Hiernach besitzen nur die Isoenzyme 1A1 und 1A3 eine messbare Aktivität hinsichtlich der ATV-Lactonisierung, wobei UGT1A3 bei höherer Substrataktivität die weitaus höhere Aktivität zeigt. Die kinetischen Parameter Michaelis-Menten Konstante Km und maximale Aktivität Vmax wurden mit rekombinantem UGT1A3 Enzym durch Messungen der Lactonisierungsaktivität (v) bei unterschiedlicher Substratkonzentration (cs) bestimmt (rechts; 2B), die Versuchsansätze und das Ergebnis für Vmax sind in Fig. 2C gezeigt.

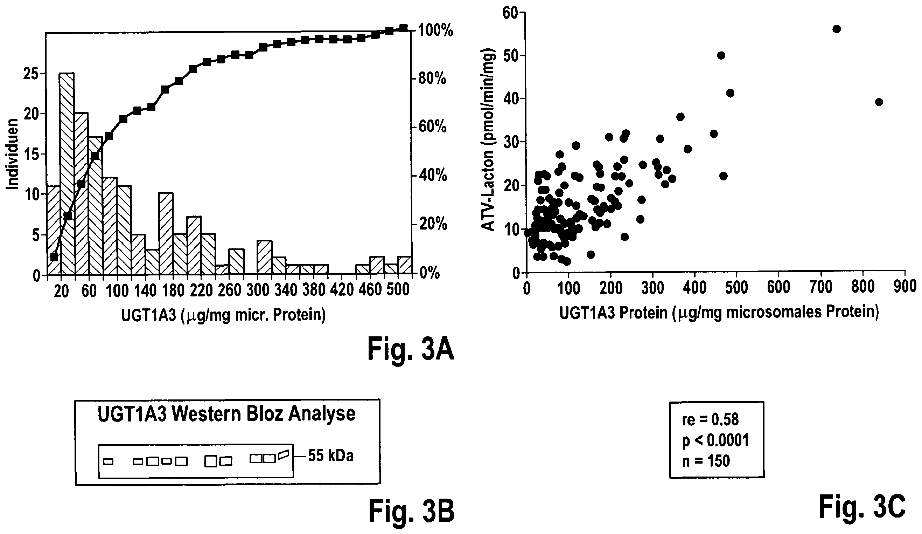

Fig. 3 zeigt links (Fig. 3A) ein Histogramm zur Verteilung des UGT1A3 Proteins in der Bevölkerungsstichprobe von 150 Leberproben der IKP-Leberbank. Unter dem Histogramm ist beispielhaft eine Western Blot Analyse gezeigt (Fig. 3B). Das vom spezifischen Antikörper markierte 55 kDa Proteinband trat als Doppelbande in Erscheinung, die durch Glykosylierung des Proteins verursacht wird. Die ansteigende Kurve im Histogramm gibt die kumulative Häufigkeit an und die geschweiften Klammern deuten mögliche Subgruppen mit erhöhter Aktivität an. Im rechten Diagramm (Fig. 3C) ist die Korrelation der Atorvastatin Lactonisierung zu den Proteinmessungen in der menschlichen Leber gezeigt. Die Korrelationen wurden statistisch ausgewertet. In der Box unter dem Diagramm ist der Spearman'sche Rang-Korrelationskoeffizient rₛ und die statistische Signifikanz als p-Wert angegeben.

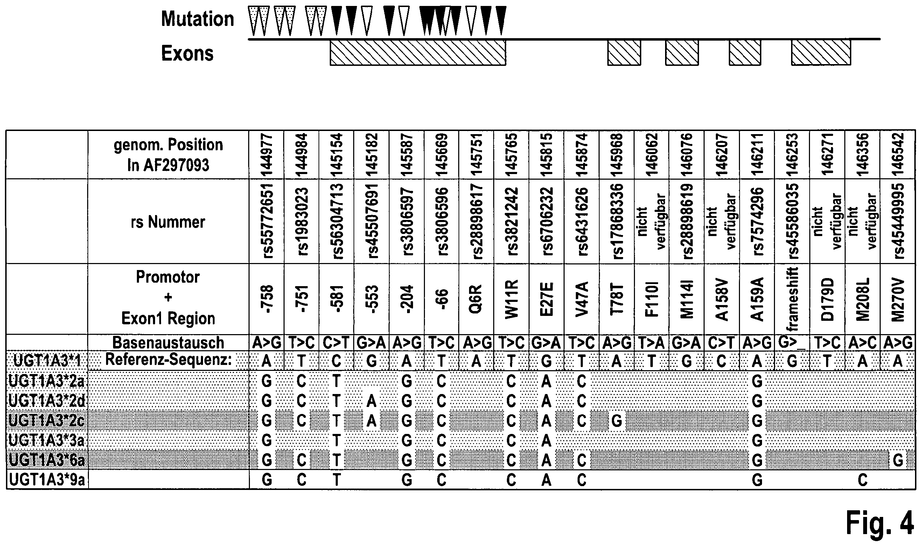

Fig. 4 zeigt eine Übersicht über die Struktur des UGT1A3 Gens mit der ungefähren Lage der in den Leberproben untersuchten Mutationen (grau: Promoterbereich; weiß: stille Mutationen im kodierenden Bereich; schwarz: Aminsäureaustausche) relativ zu den Exonen (schwarze Boxen 1-5). Darunter sind die UGT1A3 Haplotypen und Haplotyp-Varianten dargestellt, die bei den Untersuchungen in den menschlichen Leberproben identifiziert wurden. Hierzu wurden den Leberproben zugehörige DNA-Proben mittels der UGT1A3-Genotypisierungsmethode genotypisiert. Die SNP Daten (im oberen Bereich mit genomischer Position und/oder rs-Nummer angegeben) wurden anschließend mit dem Programm Phase (Version 2.1) analysiert. Im unteren Bereich ist die zu jeder untersuchten Position gehörende Base angegeben.

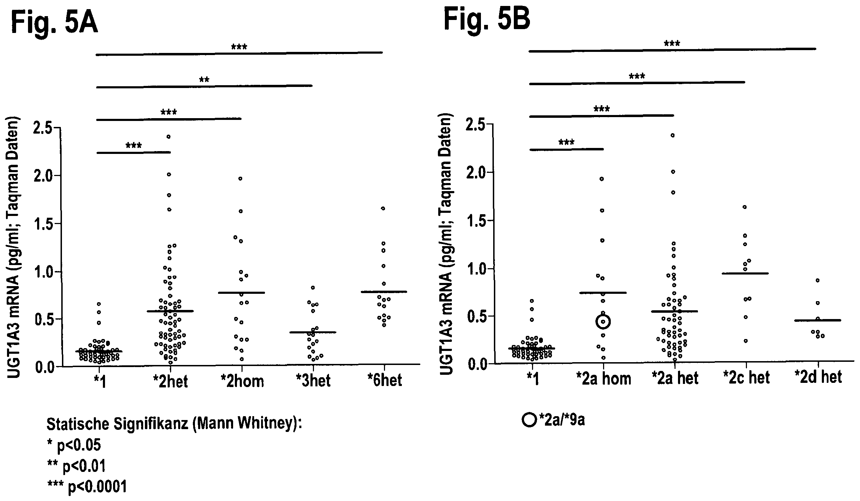

Fig. 5 zeigt die Korrelation zwischen Genotyp und Phänotyp für UGT1A3 mRNA in den untersuchten humanen Leberproben (n=150). Im linken Diagramm (Fig. 5A) ist dargestellt, welchen mRNA Wert jede Leberprobe eines bestimmten Genotyps aufweist. Das rechte Diagramm (Fig. 5B) zeigt die mRNA Werte der unterschiedlichen UGT1A3*2 Haplotypvarianten. Signifikante Unterschiede wurden mit Hilfe des "Mann Whitney Tests" bestimmt.

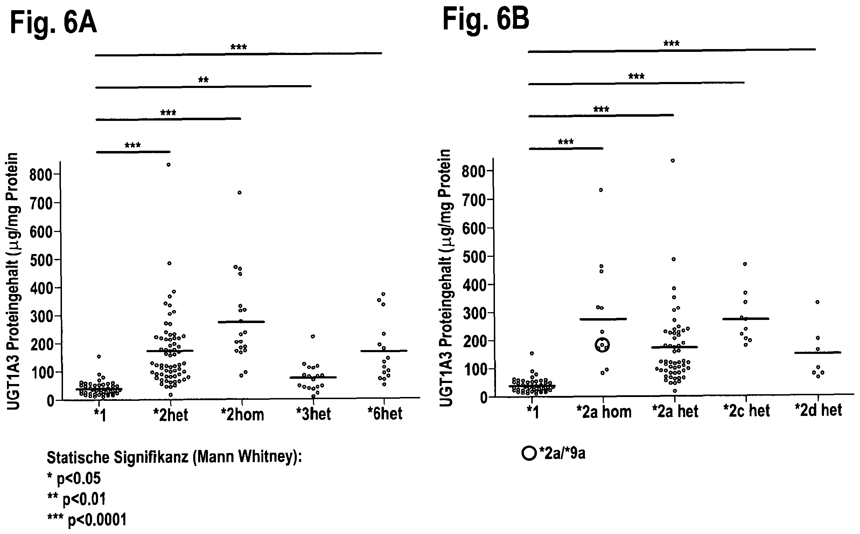

Fig. 6 zeigt die Korrelation zwischen Genotyp und Phänotyp für UGT1A3 Protein in den mikrosomalen Fraktionen der untersuchten humanen Leberproben (n=150). Im linken Diagramm (Fig. 6A) ist dargestellt, welchen UGT1A3 Protein-Wert jede Leberprobe eines bestimmten Genotyps aufweist. Das rechte Diagramm (Fig. 6B) zeigt die Protein-Werte der unterschiedlichen UGT1A3*2 Haplotypvarianten. Signifikante Unterschiede wurden mit Hilfe des "Mann Whitney Tests" bestimmt.

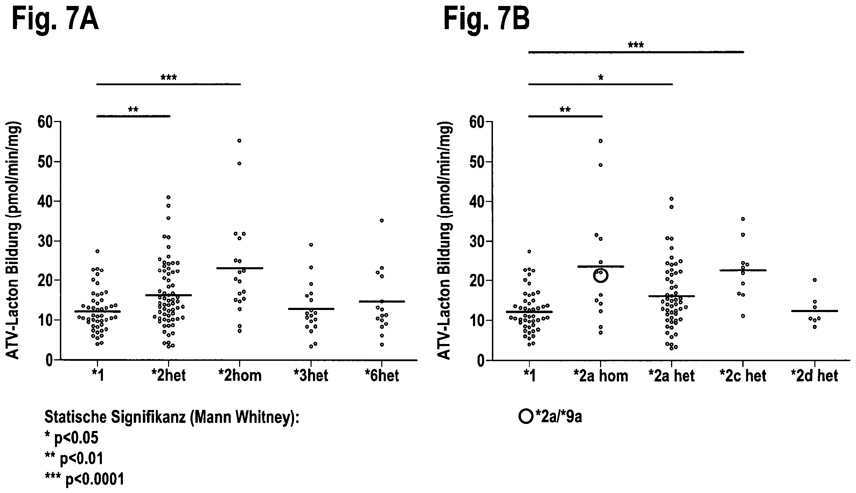

Fig. 7 zeigt die Korrelation zwischen Genotyp und Phänotyp für die Atorvastatin-Lactonisierung in den mikrosomalen Fraktionen der untersuchten humanen Leberproben (n=150). Im linken Diagramm (Fig. 7A) ist dargestellt, welchen mRNA Wert jede Leberprobe eines bestimmten Genotyps aufweist. Das rechte Diagramm (Fig. 7B) zeigt die mRNA Werte der unterschiedlichen UGT1A3*2 Haplotypvarianten. Signifikante Unterschiede wurden mit Hilfe des "Mann Whitney Tests" bestimmt.

### Material und Methoden

### Patienten DNA und Leberproben

Lebergewebe und zugehörige Blutproben gehören zu einer am IKP Stuttgart aufgebauten humanen Leberbank. Die Proben wurden in den Jahren 1999-2000 von Patienten gewonnen, die sich aus medizinischen Gründen einer leberchirurgischen Operation unterzogen (beschrieben in Wolbold et al: Sex Is a Major Determinant of CYP3A4 Expression in Human Liver; Hepatology 2003, 38:978-988). Alle Gewebeproben wurden von einem Pathologen untersucht und es wurde dadurch sichergestellt, dass nur histologisch normales Gewebe verwendet wurde. Die klinische Patientendokumentation wurde komplett anonymisiert und beinhaltet Angaben über das Geschlecht, das Alter, die medizinische Diagnose, medikamentöse Therapie, Alkohol- und Rauchgewohnheiten, und bestimmte Laborwerte. Proben von Patienten mit Hepatitis, Zirrhose oder chronischem Alkoholabusus wurden ausgeschlossen. Insgesamt 150 Leberproben, von denen qualitativ hochwertige RNA und eine komplette Dokumentation vorlagen, wurden für diese Untersuchungen verwendet. Die für Western Blot und Enzymaktivitätsmessungen verwendeten Lebermikrosomen wurden nach Standardmethoden hergestellt (beschrieben in Lang et al.: Extensive genetic polymorphism in the human CYP2B6 gene with impact on expression and function in human liver; Pharmacogenetics 2001, 11:399-415). Genomische DNA für Genotypuntersuchungen wurde aus Blutproben der jeweiligen Gewebespender isoliert. Für die Untersuchungen wurde beim örtlichen Ethikkommittee ein entsprechendes Votum eingeholt. Die Untersuchungen fanden in Übereinstimmung mit der Deklaration von Helsinki statt und alle Patienten hatten ihre schriftliche Einwilligung gegeben.

### Bestimmung der UGT1A3 mRNA

Total-RNA wurde mittels RNeasy Midi Kit (Qiagen, Hilden, Germany) aus Lebergewebe präpariert. Die Quantifzierung von UGT1A3 mRNA wurde mittels eines selbstentwickelten, spezifischen TaqMan Real-Time Reverse-Transcriptions-PCR Assay auf einem 7900HT Fast Real Time PCR System (Applied Biosystems, Foster City, CA) durchgeführt. Dazu wurden ein das Intron1 überspannendes Primerpaar 1A3_tq_neu_f/r (siehe nachstehende Tabelle 3) and die FAM-markierte UGT1A3-MGB Sonde bei Konzentrationen von 400 nmol/l bzw. 200nmol/l verwendet. Kreuzreaktivität gegenüber *UGT1A4* wurde ausgeschlossen, indem DNA Plasmide beider Gene als Template untersucht wurden (nicht gezeigte Daten). Die PCR-Reaktionsansätze enthielten 2x universal PCR Master Mix (Applied Biosystems) in einem finalen Volumen von 12.5 µl und die folgenden PCR Zykluskonditionen wurden verwendet: 50°C für 2 min; 95°C für 10 min, gefolgt von 40 Zyklen mit den Schritten 95°C für 15 sec und 60°C für 1 min.

**Tabelle 3: Amplifikationsprimer für die quantitative PCR.**

| SEQ-ID Nr. | c. Position | Primer Sequenz (5'→ 3') | Amplifications-Produkt (bp) |
|---|---|---|---|
| 28 | 347-371 | TGTTGAACAATATGTCTTTGGTCTA | 698 |
| 29 | 258-278 | GAAGGAATTTGATCGCGTTAC | 787 |
| 30 | 171-189 | GGTGGTGGTCCTCACCCTG | 874 |
| 31 | 1024-1044 | GTTCGCAAGATTCGATGGTCG | |
| 32 | 738-763 | GGATATTCTCAGTCATGCATCTGTGT | 297 |
| 33 | 1017-1034 | TTCGATGGTCGGGTTCCA | |
| 34 | 795-807 | 6FAM-CCCCAGGCCAATC-MGB | |

Die Positionen entsprechend cDNA Sequenzen ("c. Position") mit den GenBank Zugangsnummern (Acc. Numbers) CCDS2509.1 (UGT1A3), CCDS33405.1 (UGT1A4) und CCDS33404.1 (UGT1A5).

### Bestimmung des mikrosomalen UGT1A3 Proteingehalts

Das UGT1A3-Protein wurde mittels WesternBlot-Analysen aus 150 Leberproben quantifiziert. Als primärer Antikörper wurde ein monoklonaler Antikörper (ab57400 von abcam) eingesetzt. Als sekundärer Antikörper wurde Ziege-anti-Maus IRDye 800CW (LICOR Biosciences) eingesetzt, und die Detektion wurde mittels des ODYSSEY-Infrarot-Imaging-System (LICOR Biosciences) durchgeführt. 20 µg an menschlichen Lebermikrosomen wurden einer SDS-PAGE (10 % SDS-Trenngele) unterzogen und auf Nitrocellulosemembranen geblottet. Die Blots wurden mit 3 µg/ml Anti-UGT1A3 in 1 % entrahmter Milch/TBST über 2 Stunden inkubiert, die Inkubation mit dem sekundären Antikörper, 1:10.000 in 1 % entrahmter Milch (TBST) verdünnt, erfolgte anschließend 30 Minuten lang.

Die WesternBlots ergaben drei Banden bei ca. 55 kDa für die rekombinanten UGT1A3 und zwei Banden für UGT1A3 aus Leber. Nach Deglycosilierung der Proben verblieb jeweils eine Bande in beiden Fällen. Zur Quantifizierung in den Lebermikrosomen wurde die Intensität der Banden kombiniert.

Die relative Quantifizierung wurde mittels der ODYSSEY-Software gegen eine Standardkurve (5 Punkte; 0,5 - 8 µg) rekombinantem UGT1A3 (menschliche UGT1A3-Supersomes; BDBiosciences; Katalognummer 456413) durchgeführt. Die Ergebnisse aus unterschiedlichen Blots wurden auf Ergebnisse gepoolter Mikrosomen normalisiert, die bei jedem Blot eingesetzt wurden.

Die Spezifität der Antikörper wurde gegenüber 10 µg rekombinantem UGT1A1, 1A4, 1A6, 1A9, 2B4 und 2B9 getestet. Bezüglich UGT1A6, 1A9, 2B4 und 2B9 konnte keine Kreuzreaktivität gefunden werden; gegen UGT1A1 und 1A4 war bei maximaler Sensitivität eine leichte Kreuzreaktivität erkennbar, die jedoch die Datenauswertung nicht beeinträchtigte.

### Bestimmung der UGT1A3 Aktivität für Atorvastatin Lactonisierung

Zur Messung der Atorvastatin-Lacton-Bildung durch menschliche Lebermikrosomen wurde ein LC-MS Assay etabliert: Hierzu werden 25 µg Mikrosomen in 50 mM Tris HCl, pH 7,4 mit 5 mM Magnesiumchlorid, 25 µg/ml Alamethicin und 10 µM Atorvastatin in einem Gesamtvolumen von 100 µl 10 Minuten lang bei 37°C vorinkubiert. Die Reaktion wird durch Hinzufügen von 10 mM UDP-Glucuronsäure, dem Cosubstrat der UDP-Glucuronosyltransferasen (UGT), gestartet. Die Proben wurden 30 Minuten lang inkubiert. Die Reaktion wurde durch Hinzufügen von eiskaltem 25 µl 250 mM Ameisensäure in Acetonitril gestoppt und die gevortexten Proben sofort auf Eis gestellt. Um die LC/MS-Quantifizierung verfolgen zu können, wurde 10 µl interner Standard zu den 100 µl-Proben gegeben. Die Proben wurden 5 Minuten bei 13.000 rpm zentrifugiert und in Glasbehältnisse für die LC/MS-Messung gegeben.

Mit der LC/MS-Methode kann Atorvastatin, Atorvastatin-Lacton sowie deren Para- und Ortho-Hydroxy-Formen im Bereich von 5 nM bis 5 µM kombiniert quantifiziert werden. Die Proben werden auf einer XBridge Shield RP18 3,5 µm-Säule mit einer C8-Vorsäule mit einem 1 mM Ameisensäure- und Acetonitril-Gradienten 23 Minuten lang bei 30°C getrennt. Die Massen werden durch HCT Esquire plus Massenspektrometer detektiert, und zwar nach Elektrospray-Ionisierung der durch die Säule getrennten Substanzen.

### Genetische Analysen am UGT1A Lokus

Caillier et al. (2007) detektierten durch Sequenzierung 7 Promoter- und 13 Exon-1-SNPs (4 Synonyme) in 249 Patientenproben. Die UGT1A-Familie unterscheidet sich lediglich in Exon 1 und hat identische Exone 2 bis 5. Der vorliegende Ansatz bestand darin, eine Methode zu etablieren, um diese beschriebenen 18 Promoter- und Exon-1-SNPs plus 1 Frameshift-SNP in Exon 1 (rs45586035) zu detektieren. Vier Regionen, die diese SNPs enthalten, wurden mittels Qiagen HotStar Polymerase in 10 ng genomischer DNA mit Primern amplifiziert, die spezifisch für die Amplifizierung von lediglich UGT1A3 ausgebildet waren. Alle MALDI-TOF MS Amplifikationsprimer (siehe Tabelle 1 oben) hatten eine 5' "tag"-Sequenz (ACGTTGGATG) um eine effizientere Amplifikation zu ermöglichen (Empfehlung der Software MassArray Assay Design (v3.0.0). Ein spezielles PCR-Protokoll wurde verwendet, um eine hohe Spezifität zu gewährleisten, sowie um eine hohe Ausbeute zu erzielen: Multiplex PCR Ansätze in 384-well Mikrotiterplatten, Volumen 5 µL, enthielten 10 ng vorgetrocknete DNA, 4 µl "HotstarTaq Master Mix" mit HotstarTaq Polymerase (Qiagen GmbH, Hilden, Germany), 0.1 µM von jedem Amplifikationsprimer (siehe Tabelle 1 oben) und 0.5 mM MgCl₂ (Qiagen). Die PCR Bedingungen (Gene Amp PCR System 9700, Applied Biosystems, Foster City, CA) waren wie folgt: Denaturierung bei 95°C für 15 min, gefolgt von 5 Zyklen mit den Schritten 95°C für 20 sec, 65°C für 30 sec und 72°C für 1 min; gefolgt von 40 Zyklen mit den Schritten 95°C für 20 sec, 62°C für 30 sec und 72°C für 1 min; gefolgt von einem finalen Extensionsschritt bei 72°C für 10 min. Um die Spezifität zu bestätigen, wurden diese Fragmente in einer Probe sequenziert, und die PCR-Produkte einiger Proben wurden ständig auf Agarosegelen hinsichtlich korrekter Amplifizierung und mit Kontaminierungs-freien Wasserkontrollen getestet.

Nach Amplifizierung dieser Fragmente wurden überschüssige dNTPs in einem finalen Volumen von 7 µl mit 0.3 µl SAP (1.7 U/µl) in 0.17µl 10× SAP Puffer (Sequenom, San Diego, CA) and 1.53µl Wasser bei 37°C für 20 min, gefolgt von 10 min bei 85°C und 20°C für 1 sec dephosphoryliert.

Es wurden spezifische Primer entworfen, die direkt vor SNP-Positionen enden (siehe Tabelle 2 oben). Im Amplifizierungsschritt wurden sie nur um jeweils eine markierte Base verlängert, die zu der SNP-Base passt. Die Primer eines Assays wurden derart entworfen, dass deren Produkte sich in der Masse mit zumindest 15 Da unterschieden. Die detektierbaren Massen lagen im Bereich von 3000 bis 85.00 Da.

Ein spezifisches Amplifizierungsprotokoll, zusammen mit dem iPlex-Enzym sowie spezifischen Pufferkonditionen wurde für diesen Schritt eingesetzt:

Nach Zugabe von 0.2 µl iPLEX Puffer, 0.2 µL iPLEX Terminator Mix und 0.041 µl iPLEX Enzym (iPLEX Gold Reaction Kit, Sequenom) wurden 0.0112 bzw. 00224 µl Extension Primer (500µM) zugegeben, je nachdem ob die Primer Masse niedriger oder höher als 6000 Da betrug, um das Signal-Rausch-Verhältnis optimal einzustellen. Die Bedingungen der Extensionsreaktion waren wie folgt: 94°C für 30 sec, gefolgt von 40 Zyklen mit den Schritten 94°C für 5 sec, mit 5 Subzyklen von 52°C für 5 sec und 80°C für 5 sec; und einem finalen Extensionsschritt bei 72°C für 3 min.

Um störende Natrium- und Kaliumaddukte zu vermeiden, wurden die Proben entsalzt indem jeweils 6 mg Clean Resin (Sequenom) und 16 µl Wasser zugesetzt wurden. Nach 20 min Inkubation bei Raumtemperatur wurde für 20 min bei 4000 rpm zentrifugiert. Die Proben wurden dann unter Verwendung eines Nanodispensers auf 384 SpectroCHIP® Arrays (Sequenom) aufgebracht und im MassArrayTM Compact Mass Spectrometer (Sequenom) analysiert. Automatisierte Spektrenaufzeichnung wurde mit der Software Spectroacquire und Datenanalyse mit MassArray Typer Software v 3.4. durchgeführt. Nicht automatisch zugeordnete Proben wurden manuell nachanalysiert.

### Statistische und Computer-unterstützte Analysen

Die Auswertung der genetischen Daten erfolgte mittels öffentlich zugänglicher Programme. Beobachtete und erwartete Allel- und Genotyphäufigkeiten in der Leberbank Population wurden auf Abweichungen vom Hardy-Weinberg Gleichgewicht mittels des online verfügbaren DeFinetti Programms getestet (Strom TM and Wienker TF, DiFinetti Program homepage for Hardy-Weinberg equilibrium test; http://ihg.gsf.de/cgi-bin/hw/hwa1.pl). Haplotyp Berechnungen wurden mit Hilfe des PHASE Programms, Version v2.1.1. (The Matthew Stephens homepage; http://stephenslab.uchicago.edu/home.html) sowie unter Verwendung von Haploview v. 3.32 (The Broad Institute haploview homepage; http://www.broad.mit.edu/mpg/haploview/) durchgeführt.

Statistische Analysen wurden mit dem Statistik Programm Prism 4, Version 4.03 (GraphPad Software Inc.) durchgeführt. Spearman Rang-Korrelationskoeffizienten wurden zur Korrelationsanalyse der mRNA, Protein und Aktivitätsdaten bestimmt. Haplotyp-Gruppenunterschiede wurden mittels "Mann Whitney Test" bestimmt (nicht-parametrischer T-Test). Alle statistischen Tests wurden zweiseitig durchgeführt und statistische Signifikanz wurde als P < 0.05 definiert.

### Ergebnisse

Unter Einsatz von LC-MS/MS-Analytik wurde die Rolle einzelner rekombinant exprimierter CYP- und UGT-Enzyme bei der ATV-Hydroxylierung und ATV-Lactonisierung untersucht. Hierbei konnte unter Verwendung einer großen Bank für menschliches Lebergewebe mit 150 gut dokumentierten chirurgischen Leberproben das UGT-Isoenzym, das für die ATV-Lactonisierung in der Leber hauptsächlich verantwortlich ist, identifiziert werden. Ferner wurden genetische Analysen durchgeführt, um SNPs sowie Haplotypen der relevanten UGT-Gene zu identifizieren, die für die ATV-Biotransformation verantwortlich sind.

### Identifizierung von CYP3A4 als Hauptenzym für die ATV-Hydroxylierung

Unter den wichtigsten Arzneimittel-metabolisierenden CYP-Enzymen ist die Cytochrom P450 CYP3A Subfamilie mit den beiden Isoenzymen CYP3A4 und CYP3A5 die einzig relevanten P450-Enzyme, die die ATV-Hydroxylierung katalysieren. Die katalytische Aktivität von CYP3A4 war im Vergleich zu coexprimierten Cytochromen b5 mehrfach verstärkt, wobei Cytochrom b5 ein bekannter katalytischer Verstärker von CYP3A4-Biotransformationen ist.

### Identifizierung von UGT1A3 als Hauptenzym für die ATV-Lactonisierung

Fig. 2A zeigt, dass unter verschiedenen rekombinanten UGT Enzymen die UGT1A3 das Enzym mit der höchsten spezifischen Aktivität für die Katalysierung der ATV-Lactonisierung ist. UGT1A1 besaß hingegen nur eine niedrige Aktivität. Die kinetische Analyse ergab für rekombinantes UGT1A3 einen Km-Wert von 12 µM für die Bildung des Atorvastatin-Lactons (Fig. 2B und C).

UGT1A3-Proteindaten aus 150 Leberproben zeigten eine 530-fache Variabilität und eine nicht-normale Verteilung, sowie das mögliche Vorliegen von Subgruppen mit höherer Aktivität (siehe Fig. 3A). Die mikrosomale UGT1A3-Lactonisierungsaktivität zeigte eine 20-fache Variabilität innerhalb der 150 Leberproben. Die Lactonisierung korrelierte gut mit den UGT1A3-Western Blot Proteindaten (siehe Fig. 3B), und auch mit den quantitativen UGT1A3 mRNA-Daten der real-time PCR Analyse (rs = 0.38; P<0.0001; Daten nicht gezeigt).

### Genetische Analysen

Eine Auswahl an SNPs des UGT1A-Locus wurden mittels MALDI-TOF-Assays in den zu den Leberproben gehörigen DNA Proben bestimmt (n=19, siehe Fig. 4). Zusätzlich wurde separat der UGT1A1-Genotyp bestimmt. Zwischen den SNPs besteht ein Kopplungsungleichgewicht, wie mittels Haploview gezeigt werden konnte. Die Kopplung ist derart, dass Träger des UGT1A1*28 Allels in den meisten Fällen auch Träger des UGT1A3*2 Allels sind, d.h. die durch erhöhte UGT1A3 Expression bedingte erhöhte Latonisierungsfähigkeit von Atorvastatin ist auch mittels Genotypisierung für UGT1A1*28 vorherzusagen.

Analysen zur Korrelation des Genotyps mit dem Phänotyp für UGT1A3 auf der Ebene mRNA, Protein und Atorvastatin-Lactonisierung sind in den Fig. 5 bis 7 gezeigt. UGT1A3*2-, *3- und *6-Haplotypen waren die am weitesten verbreiteten Varianten der Haplotypen. Alle drei Haplotypen zeigten eine signifikant erhöhte UGT1A3-Proteinexpression (siehe Fig. 5). Die Aktivität zur ATV-Lactonisierung war bei homozygoten *2-Trägern im Vergleich zu den Wildtyp-Proben zweifach erhöht. Die Träger von *3 und *6 Haplotypen hatten keine erhöhte Lactonbildung gezeigt. Dabei ist zu berücksichtigen, dass die Probenzahl geringer war als bei den *2-Trägern und dass nur heterozygote Träger identifiziert werden konnten. Bei homozygoten Trägern könnte daher ein Effekt auf die Enzymaktivität messbar sein. Die Daten in den vorliegenden Fig. 5-7 zeigen daher klar, dass die Varianten mit einer erhöhten Funktion assoziiert sind.

## Patentansprüche

1. Verfahren zur Bestimmung einer Prädisposition eines Patienten für die Entwicklung von Muskelerkrankungen bei einer Behandlung des Patienten mit Atorvastatin, wobei in einer biologischen Probe des Patienten das Vorliegen von zumindest einem Einzelnucleotid-Polymorphismus (SNP) in einem der folgenden Haplotypen des UGT1A3 (Uridindiphosphat-Glucuronosyltransferase 1A3)-Gens bestimmt wird: UGT1A3*2 mit Varianten *2a, *2c, *2d, UGT1A3*3 oder UGT1A3*6, und/oder eine erhöhte Genexpression dieser Haplotypen des UGT1A3-Gens bestimmt wird.

2. Verfahren nach Anspruch 1, **dadurch gekennzeichnet, dass** zumindest einer der folgenden SNPs im UGT1A3-Gen bestimmt wird: rs55772651, rs1983023, rs56304713, rs45507691, rs3806597, rs3806596, rs3821242, rs6706232, rs6431625, rs17868336, rs7574296, g.146356 (gen. Pos. in AF297093), rs45449995

3. Verfahren nach Anspruch 1, **dadurch gekennzeichnet, dass** zumindest ein mit den UGT1A3*2 Haplotypen in Kopplungsungleichgewicht befindlicher SNP im UGT1A1*28 Haplotyp bestimmt wird.

4. Verfahren nach einem der Ansprüche 1 bis 3, **dadurch gekennzeichnet, dass** die erhöhte Genexpression der UGT1A3-Haplotpyen über einen erhöhten mRNA-Level, einen erhöhten Protein-Level und/oder Enzymaktivitäts-Level bestimmt wird.

5. Verfahren nach einem der Ansprüche 1 bis 4, **dadurch gekennzeichnet, dass** zum Nachweis des zumindest einen SNP ein Oligonucleotid eingesetzt wird, das ausgewählt ist aus den Oligonucleotiden mit den SEQ-ID Nrn. 1 bis 27 aus dem beigefügten Sequenzprotokoll.

6. Verfahren nach einem der Ansprüche 1 bis 5, **dadurch gekennzeichnet, dass** zur Bestimmung des zumindest einen SNPs zumindest eines der folgenden Verfahren eingesetzt wird: PCRbasierte Verfahren, DNA-Sequenzierverfahren, Hybridisierungsverfahren, Massenspektroskopie, HPLC-Verfahren, Primer-Extension-Verfahren.

7. Verfahren nach einem der Ansprüche 1 bis 6, **dadurch gekennzeichnet, dass** die Muskelerkrankung ausgewählt ist aus Myopathie und Rhabdomyelose.

8. Verwendung eines Kits zur Bestimmung einer Prädisposition eines Patienten für die Entwicklung von Muskelerkrankungen bei einer Behandlung des Patienten mit Atorvastatin, wobei das Kit zumindest ein Oligonucleotid aufweist, das ausgewählt ist aus zumindest einem der Oligonucleotide mit den SEQ-ID-Nrn. 1 bis 10, 13, 14, 15, 17 bis 21, sowie 24 bis 27 aus dem beigefügten Sequenzprotokoll.

9. Verwendung von zumindest einem SNP in einem der folgenden Haplotypen UGT1A3*2 mit Varianten *2a, *2c, *2d, UGT1A3*3 oder UGT1A3*6, im menschlichen UGT1A3-Gen zur Bestimmung der Prädisposition eines Patienten zur Entwicklung von Muskelerkrankungen und/oder zu veränderter Biotransformation bei der Behandlung von Atorvastatin.

10. Verwendung nach Anspruch 9, **dadurch gekennzeichnet, dass** der SNP ausgewählt ist aus zumindest einem der folgenden: rs55772651, rs1983023, rs56304713, rs45507691, rs3806597, rs3806596, rs3821242, rs6706232, rs6431625, rs17868336, rs7574296, g.146356 (gen. Pos. in AF297093), rs45449995.

11. Verwendung nach Anspruch 9, **dadurch gekennzeichnet, dass** der SNP ausgewählt ist aus mit den UGT1A3*2 Haplotypen in Kopplungsungleichgewicht befindlichen SNPs im UGT1A1*28 Haplotyp.

## Claims

1. Method for determining a predisposition of a patient for the development of muscle diseases in a treatment of the patient with atorvastatin, wherein, in a biological sample from the patient, the presence of at least one single nucleotide poly-morphism (SNP) in one of the following haplotypes of the UGT1A3 (uridine diphosphate glucuronosyl transferase 1A3) gene is determined: UGT1A3*2 with variants *2a, *2c, *2d, UGT1A3*3 or UGT1A3*6, and/or an increased gene expression of these haplotypes of the UGT1A3 gene is determined.

2. Method according to Claim 1, **characterized in that** at least one of the following SNPs in the UGT1A3 gene is determined: rs55772651, rs1983023, rs56304713, rs45507691, rs3806597, rs3806596, rs3821242, rs6706232, rs6431625, rs17868336, rs7574296, g.146356 (gen. Pos. in AF297093) and rs45449995.

3. Method according to Claim 1, **characterized in that** at least one SNP which is in linkage disequilibrium with the UGT1A3*2 haplotypes in the UGT1A1*28 haplotype is determined.

4. Method according to one of Claims 1 to 3, **characterized in that** the increased gene expression of the UGT1A3 haplotypes is determined via an increased mRNA level, an increased protein level and/or enzyme activity level.

5. Method according to one of Claims 1 to 4, **characterized in that** for the detection of the at least one SNP, an oligonucleotide is used which is selected from the oligonucleotides with the SEQ ID Nos. 1 to 27 from the attached sequence protocol.

6. Method according to one of Claims 1 to 5, **characterized in that** for the determination of the at least one SNP, at least one of the following methods is used: PCR-based methods, DNA sequencing methods, hybridization methods, mass spectroscopy, HPLC methods and primer extension methods.

7. Method according to one of Claims 1 to 6, **characterized in that** the muscle disease is selected from myopathy and rhabdomyelosis.

8. Use of a diagnostic kit for the determination of a predisposition of a patient for the development of muscle diseases in a treatment of the patient with atorvastatin, wherein the kit contains at least one oligonucleotide which is selected from at least one of the oligonucleotides with the SEQ ID Nos. 1 to 10, 13, 14, 15, 17 to 21, and 24 to 27 from the attached sequence protocol.

9. Use of at least one SNP in one of the following haplotypes UGT1A3*2 with variants *2a, *2c, *2d, UGT1A3*3 or UGT1A3*6, in the human UGT1A3 gene for the determination of the predisposition of a patient to the development of muscle diseases and/or to changed biotransformation in the treatment with atorvastatin.

10. Use according to Claim 9, **characterized in that** the SNP is selected from at least one of the following: rs55772651, rs1983023, rs56304713, rs45507691, rs3806597, rs3806596, rs3821242, rs6706232, rs6431625, rs17868336, rs7574296, g.146356 (gen. Pos. in AF297093) and rs45449995.

11. Use according to Claim 9, **characterized in that** the SNP is selected from the SNPs which are in linkage disequilibrium with the UGT1A3*2 haplotypes in the UGT1A1*28 haplotype.

## Revendications

1. Procédé de détermination d'une prédisposition d'un patient au développement de maladies musculaires lors d'un traitement du patient avec de l'atorvastatine, selon lequel la présence d'au moins un polymorphisme nucléotidique simple (SNP) dans un des haplotypes suivants du gène UGT1A3 (uridindiphosphateglucuronosyltransférase 1A3) est déterminée dans un échantillon biologique du patient : UGT1A3*2 avec les variantes *2a, *2c, *2d, UGT1A3*3 ou UGT1A3*6, et/ou une expression génique accrue de ces haplotypes du gène UGT1A3.

2. Procédé selon la revendication 1, **caractérisé en ce qu'**au moins un des SNP suivants dans le gène UGT1A3 est déterminé : rs55772651, rs1983023, rs56304713, rs45507691, rs3806597, rs3806596, rs3821242, rs6706232, rs6431625, rs17868336, rs7574296, g.146356 (pos. gén. dans AF297093), rs45449995.

3. Procédé selon la revendication 1, **caractérisé en ce qu'**au moins un SNP dans l'haplotype UGT1A1*28 se trouvant en déséquilibre de liaison avec les haplotypes UGT1A3*2 est déterminé.

4. Procédé selon l'une quelconque des revendications 1 à 3, **caractérisé en ce que** l'expression génique accrue des haplotypes d'UGT1A3 est déterminée par un niveau d'ARNm accru, un niveau de protéines accru et/ou un niveau d'activité enzymatique accru.

5. Procédé selon l'une quelconque des revendications 1 à 4, **caractérisé en ce qu'**un oligonucléotide qui est choisi parmi les oligonucléotides ayant les SEQ ID n° 1 à 27 du protocole de séquences joint est utilisé pour la détection dudit au moins un SNP.

6. Procédé selon l'une quelconque des revendications 1 à 5, **caractérisé en ce qu'**au moins un des procédés suivants est utilisé pour la détermination dudit au moins un SNP: les procédés à base de PCR, les procédés de séquençage d'ADN, les procédés d'hybridation, la spectroscopie de masse, les procédés HPLC, les procédés d'extension d'amorce.

7. Procédé selon l'une quelconque des revendications 1 à 6, **caractérisé en ce que** la maladie musculaire est choisie parmi une myopathie et une rhabdomyélose.

8. Utilisation d'un kit pour la détermination d'une prédisposition d'un patient au développement de maladies musculaires lors d'un traitement du patient avec de l'atorvastatine, le kit comprenant au moins un oligonucléotide, qui est choisi parmi au moins un des oligonucléotides ayant les SEQ ID n° 1 à 10, 13, 14, 15, 17 à 21, ainsi que 24 à 27 du protocole de séquences joint.

9. Utilisation d'au moins un SNP dans un des haplotypes suivants : UGT1A3*2 avec les variantes *2a, *2c, *2d, UGT1A3*3 ou UGT1A3*6 dans le gène UGT1A3 humain pour la détermination de la prédisposition d'un patient au développement de maladies musculaires et/ou à une biotransformation modifiée lors du traitement avec de l'atorvastatine.

10. Utilisation selon la revendication 9, **caractérisée en ce que** le SNP est choisi parmi au moins un des suivants : rs55772651, rs1983023, rs56304713, rs45507691, rs3806597, rs3806596, rs3821242, rs6706232, rs6431625, rs17868336, rs7574296, g.146356 (pos. gén. dans AF297093), rs45449995.

11. Utilisation selon la revendication 9, **caractérisée en ce que** le SNP est choisi parmi les SNP dans l'haplotype UGT1A1*28 se trouvant en déséquilibre de liaison avec les haplotypes UGT1A3*2.
